# EUROPÄISCHE PATENTSCHRIFT

(11) **EP 0 448 924 B1**
(45) Veröffentlichungstag und Bekanntmachung des Hinweises auf die Patenterteilung: **06.07.1994**
(21) Anmeldenummer: 90890336.2
(22) Anmeldetag: 18.12.1990
(51) Int. Cl.: B01D 61/02, C07D 295/023, C07D 295/24, C02F 1/44, D01F 13/02

(54) **Verfahren zum Abtrennen von Wasser aus einer verdünnten, wässerigen Lösung von N-Methylmorpholin-N-Oxid, N-Methylmorpholin und/oder Morpholin**
Process for separating water from a diluted aqueous solution of N-methylmorpholine-N-oxide, N-methylmorpholine and/or morpholine
Procédé de séparation de l'eau d'une solution aqueuse diluée de N-méthylmorpholine-N-oxyde, N-méthylmorpholine et/ou morpholine

(30) Priorität: 28.03.1990 AT 722/90
(43) Veröffentlichungstag der Anmeldung: 02.10.1991
(73) Patentinhaber: LENZING AKTIENGESELLSCHAFT, 4860 Lenzing (AT)
(72) Erfinder: Zikeli, Stefan, A-4844 Regau (AT); Weinzierl, Karin, Dr., A-4850 Timelkam (AT); Eichinger, Dieter, Dr., A-4840 Vöcklabruck (AT); Astegger, Stefan, Dr., A-4840 Vöcklabruck (AT); Wolschner, Bernd, Dr., A-4840 Vöcklabruck (AT); Firgo, Heinrich, Dr., A-4840 Vöcklabruck (AT)
(74) Vertreter: Müllner, Erwin, Dr.

(56) Entgegenhaltungen:
- SU-A- 1 427 011
- CHEMIE-INGENIEUR-TECHNIK, Band 61, Nr. 7, Juli 1989, Seiten 535-544, Weinheim, DE; R. RAUTENBACH et al.: "Membranverfahren zur Fraktionierung von Gemischen mit organischen Komponenten"
- DESALINATION, Band 71, 1989, Seiten 107-126, Amsterdam, NL; J. JIANG et al.: "Study on the interaction between membranes and organic solutes by the HPLC method"
- ENVIRONMENTAL SCIENCE & TECHNOLOGY, Band 10, Nr. 4, April 1976, Seiten 364-369, Easton, US; H.H.P. FANG et al.: "Reverse osmosis separation of polar organic compounds in aqueous solution"

## Beschreibung

Die vorliegende Erfindung betrifft ein Verfahren zum Abtrennen von Wasser aus einer bei der Herstellung cellulosischer Formkörper mit Hilfe des Lösungsmittels N-Methylmorpholin-N-Oxid anfallenden verdünnten wässerigen Lösung von N-Methylmorpholin-N-Oxid, N-Methylmorpholin, Morpholin oder deren Mischungen.

Mit anderen Worten: die verdünnte wässerige Lösung soll aufkonzentriert werden.

Zur Lösung von Cellulose kann als Lösungsmittel eine Mischung von N-Methylmorpholin-N-Oxid (im folgenden NMMO genannt) und Wasser eingesetzt werden. Die gelöste Cellulose kann extrudiert oder versponnen werden und anschließend z. B. mit Hilfe von Wasser ausgefällt werden, um Filme, Fäden oder Formteile auf Cellulosebasis zu erhalten. Dies ist aus der AT-B-376 986 bekannt.

Zur Lösung der Cellulose wird ein Gemisch aus Cellulose, Wasser und NMMO unter Vakuum gerührt und temperiert. Dabei wird solange Wasser abgezogen, bis die Cellulose vollständig gelöst ist. (Siehe Beispiel XII der AT-B-376 986.) Der anfallende Wasserdampf wird in einem Kondensator kondensiert. Infolge der thermischen Belastung entstehen - abhängig von Temperatur, Dauer der thermischen Belastung, Cellulosekonzentration und der Qualität des NMMO - N-Methylmorpholin und Morpholin. Beide Substanzen sind flüchtig und werden mit dem Wasserdampf als Kopfprodukt kondensiert (sog. Brüdenkondensat). Außerdem reißen Spritzverluste und Siedeverzüge NMMO mit.

Im Brüdenkondensat können bis maximal 5% an N-Methylmorpholin und Morpholin bzw. NMMO nachgewiesen werden. Das anfallende Brüdenkondensat kann aufgrund des Gewässerschutzes nicht abgeleitet werden; außerdem würde dies einen Chemiekalienverlust bedeuten.

Wegen des Gewässerschutzes muß daher das N-Methylmorpholin, das Morpholin und das NMMO aus dem Brüdenkondensat abgetrennt werden. Durch Destillation bzw. Rektifikation ist dies nicht oder jedenfalls nur mit großem Aufwand möglich; diese Möglichkeit scheidet daher aus wirtschaftlichen Überlegungen aus.

Anderseits enthält das Fällbad, in dem die Cellulose beim NMMO-Prozeß ausgefällt wird, NMMO. Soll dieses NMMO zur Lösung von weiterer Cellulose verwendet werden, so muß das Fällbad aufkonzentriert werden. Geschieht dies durch Eindampfen, so entstehen wiederum N-Methylmorpholin und Morpholin. Es besteht somit Bedarf an einer Alternative zum Eindampfen, um stark verdünnte bei der Herstellung cellulosischer Formkörper mit Hilfe des Lösungsmittel NMMO anfallende Lösungen von NMMO, N-Methylmorpholin, Morpholin und deren Mischungen aufzukonzentrieren.

Eine Abtrennung könnte z.B. durch Verwendung von Kationentauscherharzen erfolgen. Dies ist für das Fällbad aus SU-A-1 427 011 bekannt. Nach erfolgter Beladung des Austauschermaterials muß aber mit anorganischen oder organischen Säurenregeneriert werden. Dabei ergeben sich folgende Nachteile:
Bei Verwendung von anorganischen und organischen Säuren zur Regenerierung ist mit einer starken Abwasserbelastung durch Regeneriersäuren und Spülflüssigkeiten zu rechnen; die Regeneration mit organischen Säuren läßt zwar eine Kreislaufführung der Regeneriersäure und eine anschließende vollständige Entsorgung durch Verbrennung zu, die zu entfernenden Amine entgehen aber einer Aufarbeitung und Recyclierung, da sie als Salze vorliegen (Chemikalienverluste beim Prozeß); sowohl die Investitionskosten als auch die Betriebskosten einer Ionenaustauscheranlage sind relativ hoch.

Es besteht somit Bedarf an einer verbesserten Abtrennung von N-Methylmorpholin, Morpholin, NMMO und deren Mischungen aus bei der Herstellung cellulosischer Formkörper mit Hilfe des Lösungsmittel NMMO anfallenden wässerigen Lösungen.

Schließlich ist noch festzustellen, daß beim NMMO-Prozeß Verunreinigungen durch Avivage auftreten können, die auch abgetrennt werden müssen.

Es ist daher eine Aufgabe der vorliegenden Erfindung, ein Verfahren zu schaffen, bei dem aus einer bei der Herstellung cellulosischer Formkörper mit Hilfe des Lösungsmittel NMMO anfallenden verdünnten wässerigen Lösung von NMMO, N-Methylmorpholin, Morpholin oder deren Mischungen sowie gegebenenfalls Avivage relativ reines Wasser abgetrennt werden kann, sodaß die Lösung aufkonzentriert wird, ohne daß die gelösten Stoffe verändert werden. Die Investitions- und die Betriebskosten dieses Verfahrens sollen niedrig sein.

Diese Aufgaben werden durch ein Verfahren der eingangs genannten Art erfindungsgemäß dadurch gelöst, daß die Lösung in einer Umkehrosmoseanlage mit einem Druck, der größer als der osmotische Druck ist, durch eine semipermeable Membran gepreßt wird.

Die Membran hält die gelösten Stoffe weitestgehend zurück.

Membranverfahren, insbesondere die Umkehrosmose, sind allgemein in CHEMIE-INGENIEUR-TECHNIK, Band 61, Nr.7, Juli 1989, S. 535-544 beschrieben. Es heißt dort, daß das Rückhaltevermögen sehr stark konzentrationsabhängig ist und bei manchen Bedingungen sogar negative Werte annehmen kann. (S. 539, Spalte 2, 2.Absatz), und daß der Einfluß der molekularen Struktur organischer Verbindungen auf die Selektivität der Umkehrosmosemembranen sehr wesentlich ist (Seite 539, Spalte 2, letzter Absatz).

Bei der vorliegenden Anwendung liegen besonders gut geeignete Werte für den Druck bei 40 bar, für die Temperatur zwischen 25 und 75°C.

Das durch die Membran gepreßte Wasser ist sehr rein. Es kann ohne Bedenken in die Kanalisation abgelassen oder in den Prozeßkreislauf zurückgeführt werden. Durch die zuletzt genannte Möglichkeit wird natürlich Frischwasser gespart. Die von der Membran zurückgehaltenen gelösten Stoffe liegen relativ konzentriert vor, da durch das erfindungsgemäße Verfahren die ursprüngliche Konzentration stark erhöht wird.

Es ist daher zweckmäßig, wenn danach die aufkonzentrierte Lösung in an sich bekannter Weise zu N-Methylmorpholin-N-Oxid weiterverarbeitet wird. Auf diese Weise wird der Verbrauch von NMMO verringert und eine Umweltbelastung vermieden, weil alle abgetrennten bzw. aufkonzentrierten Stoffe nach entsprechender Aufarbeitung als NMMO in den Prozeßkreislauf zurückgeführt werden können.

Durch die Erfindung wird erstmals ermöglicht, eine vollständig abwasserfreie Prozeßführung bei der Herstellung cellulosischer Formkörper mit Hilfe des Lösungsmittels NMMO zu realisieren.

Durch diese Kreislaufschließung kann die Wirtschaftlichkeit des Prozesses wesentlich erhöht werden.

Durch Verfahren, die aus den im folgenden angeführten Schriften bekannt sind, kann N-Methylmorpholin zu NMMO aufoxidiert werden. Morpholin wird in einem anschließenden Schritt abdestilliert. Das abdestillierte Morpholin wird methyliert und anschließend ebenfalls zu NMMO oxidiert. Das erhaltene NMMO wird in den Prozeß zurückgeführt.

Schriften, in denen Verfahren zur Herstellung von NMMO aus N-Methylmorpholin beschrieben sind:
GB-A-1 144 048 (EASTMAN KODAK COMPANY), Beispiel 1
DD-A- 246 997 (VEB CHEMIEFASERKOMBINAT SCHWARZA "WILHELM PIECK")
DE-C-36 18 352 (HÜLS AG)

Schriften, in denen Verfahren zur Herstellung von N-Methyl-Morpholin aus Morpholin beschrieben sind:
DE-A-32 09 675 (TEXACO DEVELOPMENT CORP.)
DE-A-37 18 388 (TEXACO DEVELOPMENT CORP.)
DE-C-35 04 899 (HÜLS AG)

Im folgenden wird unter Bezugnahme auf die beiliegende Zeichung eine Vorrichtung beschrieben, die zur Durchführung der vorliegenden Erfindung geeignet ist.

Das abzutrennende Brüdenkondensat, welches beim NMMO-Prozeß - wie oben beschrieben - entsteht, wird von der Zufuhrpumpe 1 über eine Leitung 2 in den Zirkulationsbehälter oder Retentatbehälter 3 gefüllt.

Die im Retentatbehälter 3 enthaltene Flüssigkeit wird von einer Vordruckpumpe 4, z.B. einer Kreiselpumpe, einem Wärmetauscher 5 zugeführt, wo die Flüssigkeit auf eine bestimmte Temperatur gebracht wird. Von dort gelangt sie über ein Filter 6 zu einer oder mehreren Hochdruckpumpen 7, die die temperierte Flüssigkeit unter ausreichend hohen Druck (höher als der osmotische Druck) setzen. Die unter Druck stehende Flüssigkeit wird einem Membranmodul 8 zugeführt, durch dessen Membrane nur nahezu reines Wasser gelangen kann (Permeat). Die gelösten Stoffe (NMMO, N-Methylmorpholin und Morpholin) werden zurückgehalten (Retentat). Das Retentat wird über eine Leitung 9 zurück zum Retentatbehälter 3 geleitet. Das permeat wird kontinuierlich über eine Leitung 10 abgezogen und kann z.B. dem Permeatbehälter 12 zugeführt werden. Von dort kann es als Prozeßwasser dem Prozeß wieder zugeführt werden.

Mit Hilfe des Ventils V1 nach der Hochdruckpumpe und des Ventils V2 nach dem Membranmodul kann die Anströmung bzw. der notwendige Systemdruck eingestellt werden. Die Membranmodule, welche in Serie und/oder parallel geschaltet sein können, sind so gewählt, daß sie den Betriebstemperatur- und und Druckbereich sowie die chemische Beständigkeit abdecken. In Abhängigkeit vom Produkt, dem Trenneffekt und der geforderten Retentatkonzentration kann die Anlage diskontinuierlich oder kontinuierlich arbeiten. Beim diskontinuierlichen Betrieb wird das Brüdenkondensat durch die Membranmodule 8 gepumpt und das austretende Retentat wieder in den Retentatbehälter 3 zurückgeführt. Dadurch steigt die Aminkonzentration im Retentatbehälter 3 bis zur geforderten Endkonzentration an. Die so erhaltenen hochkonzentrierten Lösungen werden anschliessend, wie oben beschrieben, weiterverarbeitet und in den Prozeß zurückgeführt. Wird bei der erreichten Endkonzentration dem Retentatbehälter 3 frisches Brüdenkondensat zugeführt, so kann unter gleichzeitigem Abstoß von Retentat (Ventil V3, Leitung 11) der Prozeß kontinuierlich betrieben werden.

Es werden also durch Anwendung der Umkehrosmosetechnik die gelösten Moleküle auf physikalischer Basis mit Hilfe von Membranen abgetrennt. Die quantitative Rückhaltung und Aufkonzentrierung mittels Umkehrosmoseverfahrens geschieht dadurch, daß das verunreinigte Brüdenkondensat kontinuierlich im Kreislauf geführt wird, wobei gleichzeitig das aminfreie Permeat abgezogen wird. Das so erhaltene Konzentrat (Retentat) kann, wie oben beschrieben, weiterverwendet werden; damit reduzieren sich die Lösungsmittelverluste beim NMMO-Prozeß, und die Wirtschaftlichkeit dieses Verfahrens kann dadurch gesteigert werden. Das anfallende Permeat kann bedenkenlos in das Kanalsystem abgeleitet werden. Es kann aber auch in den Produktionsprozeß zurückgeführt werden, wodurch teures Frischwasser eingespart werden kann. Der gewünschte Trenneffekt wird somit ohne zusätzliche Chemiekalien erreicht.

Durch Rückführung des Retentates und Verwendung des Permeates an Stelle von Frischwasser kann der Lösungsmittelkreislauf vollständig geschlossen werden.

Durch die hervorragenden Eigenschaften und die äußerst wirtschaftliche Betriebsweise (Chemikalienrückführung) des Umkehrosmoseverfahrens kann dem Gewässerschutz sowie dem wirtschaftlichen Gestalten des NMMO-Prozesses Rechnung getragen werden.

Im folgenden werden Beispiele für das erfindungsgemäße Verfahren angeführt, die mit der eben beschriebenen Vorrichtung durchgeführt wurden:

### BEISPIEL 1

550 l Brüdenkondensat mit einer Aminkonzentration von 0,07 % (das Verhältnis von N-Methylmorpholin zu Morpholin war 1:1) wurden mit der Zufuhrpumpe 1 über die Rohrleitung 2 dem Retentatbehälter 3 zugeführt. Mittels der Vordruckpumpe 4 wurde über einen Wärmetauscher 5, der für die Einhaltung der Betriebstemperatur von 30°C sorgte, und das Filter 6 die Hochdruckpumpen 7 kontinuierlich angespeist.

Über die Ventile V1 und V2 wurde ein Betriebsdruck von 40 bar und eine Überströmung des Membranmoduls von 2,5 m³/h eingestellt. Verwendet wurden Wickelmodule des Typs FILMTEC SW30 HR4040 (FILMTEC ist ein eingetragenes Warenzeichen) aus Polysulfon der Firma Dow Chemicals mit einem Rückhaltevermögen für Meerwasser von 99,5 %.

Das gereinigte Permeat wurde kontinuierlich über die Leitung 10, das Retentat solange über die Leitung 9 in den Retentatbehälter 3 rückgegührt, bis die gewünschte Aufkonzentrierung erreicht war. Dann wurde über V3 zur Weiterverarbeitung abgestoßen.

Alle 10 Minuten wurden der Permeatfluß bestimmt und Proben des Retentats zur Konzentrationsbestimmung gezogen. Zu Beginn der Trennung lag der Permeatfluß bei 41,1 l/m²/h und nahm während der Aufkonzentrierung auf 29,2 l/m²/h ab. Die Retentatkonzentration lag bei 2,1 %, es wurde also eine Aufkonzentrierung um einen Faktor 30 erreicht.

Die Aminkonzentration im Permeat lag zwischen 0,0004 % und 0,0011 %, es wurde also ein Rückhaltevermögen der Membran gegenüber N-Methylmorpholin und Morpholin von 99,5 bis 99,9,%, bezogen auf die Retentatkonzentration,(98,6 bis 99,5%, bezogen auf die Ausgangskonzentration) erreicht.

### BEISPIEL 2

550 l Kondensat (Konzentration 0,09 %) wurden mittels der Vordruckpumpe 4 über den Wärmetauscher 5, der für die Einhaltung einer Temperatur von 40°C sorgte, und das Filter 6 den Hochdruckpumpen 7 zugeführt.

Es wurde ein Betriebsdruck von 40 bar und eine Überströmung der Membran von 1,25 l/m²/h eingestellt. Verwendet wurde eine Wickelmembran des Typs SW30 HR4040 (Firma Dow Chemicals).

Das gereinigte Permeat (Konzentration 0,0009 bis 0,042 %) wurde über die Leitung 10 kontinuierlich abgezogen, das Retentat solange im Kreis geführt, bis eine Konzentration von 10,3 % erreicht war. Es wurde eine Aufkonzentrierung der Amine auf das 114-fache und damit eine Trennwirkung der Membran von 99 % bis 99,6 %, bezogen auf die Retentatkonzentration, erreicht. Der Permeatfluß lag zu Anfang bei 54,8 l/m²/h und fiel mit fortschreitender Aufkonzentrierung auf 5,5 l/m²/h ab.

### BEISPIEL 3

Mit der Vordruckpumpe 4 wurden 550 l Brüdenkondensat (Ausgangskonzentration 0,12 % Amin, das Verhältnis von N-Methylmorpholin zu Morpholin war 1:1) über den Wärmetauscher 5 (Arbeitstemperatur 40°C) und den Hochdruckpumpen 7 dem Membran-modul 8 (Filmtec eingetragenes Warenzeichen Wickelmembran der Type SW30 HR4040) zugeführt. Die Überströmung der Membran lag bei 2,5 m³/h, der Betriebsdruck war 40 bar. Durch Kreislaufführung des Retentats erreichte jenes eine Konzentration von 11 %, das kontinuierlich abgezogene Permeat hatte eine Aminkonzentration zwischen 0,0004 % und 0,06 %. Damit lag das Rückhaltevermögen der Membran bei 99,5 % bzw. bei 99,7 % (bezogen auf Retentatkonzentration). Der Permeatfluß verringerte sich von 57,8 l/m²/h auf 7,5 l/m²/h. Die erreichte Aufkonzentrierung der Amine war Faktor 92.

### BEISPIEL 4

Mit der Vordruckpumpe 4 wurden 550 l Waschwasser aus der letzten Stufe des NMMO-Prozesses mit einer NMMO-Konzentration von 0,05 % dem Wärmetauscher, der für eine konstante Betriebstemperatur von 40°C sorgte, zugeführt. Die Hochdruckpumpen 7 sorgten für einen Betriebsdruck von 40 bar. Zur Aufkonzentrierung des NMMO wurde eine Filmtecmembran (FILMTEC ist ein eingetragenes Warenzeichen) der Firma DOW Chemicals, Type SW30 HR4040, verwendet. Die Überströmung der Membran lag bei 2500 l/h. Das Retentat wurde in den Retentatbehälter 3 rückgeführt, vom kontinuierlich abgezogenen Permeat wurde alle 15 Minuten der Fluß bestimmt. Die Endkonzentration des Retentats war 6,5 %, im Permeat konnte bis zu einer Nachweisgrenze von 10 ppm am HPLC kein NMMO nachgewiesen werden. Der Permeatfluß lag während der Trennung zwischen 59,1 l/m²/h und 24,2 l/m²/h. Es erfolgte eine Aufkonzentrierung auf das 130-fache.

### BEISPIEL 5

1400 l Abwasser mit einer Ausgangskonzentration von 0,01% NMMO und 0,02% Avivage wurden mit der Vordruckpumpe 4 über den Wärmetauscher 5, der für die Einhaltung einer Temperatur von 30°C sorgte, und das Filter 6 den Hochdruckpumpen 7 zugeführt. Es wurde ein Betriebsdruck von 40 bar und eine Überströmung der Membran von 1,25 m³/h eingestellt. Verwendet wurden Wickelmodule der Fa.Dow Chemicals des Typs SW 30HR4040.
Das gereinigte Permeat wurde über die Leitung 10 kontinuierlich abgezogen, das Retentat solange im Kreis geführt, bis eine Konzentration an NMMO von 93 g/l erreicht war. Das entspricht einer Aufkonzentrierung, bezogen auf NMMO, um das 930 fache. Bis zu einer Aufkonzentrierung um das ca. 600 fache war kein NMMO im Permeat nachweisbar, Avivage konnte auch bei der höchsten Anreicherung des Retentats im Permeat nicht nachgewiesen werden. Das Rückhaltevermögen der Membran bezüglich NMMO lag bei 99.89%. Der Permeatfluß lag zu Beginn der Trennung bei 44,4 l/m²/h und fiel mit zunehmender Retentatkonzentration auf 4,9 l/m²/h.

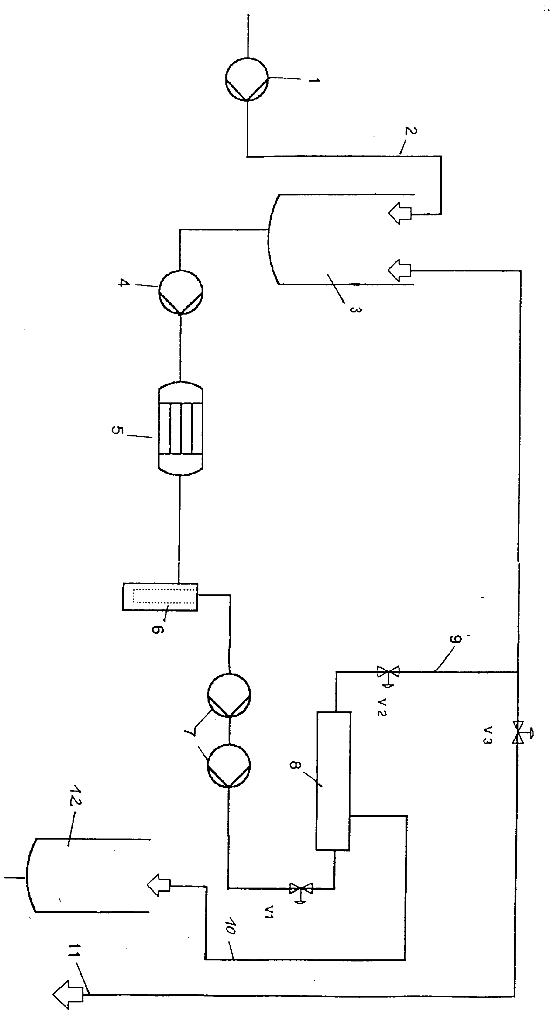

## Patentansprüche

1. Verfahren zum Abtrennen von Wasser aus einer bei der Herstellung cellulosischer Formkörper mit Hilfe des Lösungsmittels N-Methylmorpholin-N-Oxid anfallenden verdünnten wässerigen Lösung von N-Methylmorpholin-N-Oxid, N-Methylmorpholin, Morpholin oder deren Mischungen, wobei die Lösung in einer Umkehrosmoseanlage mit einem Druck, der größer als der osmotische Druck ist, durch eine semipermeable Membran gepreßt wird.

2. Verfahren nach Anspruch 1, bei dem die Lösung mit einem Druck von 40 bar durch die semipermeable Wand gepreßt wird.

3. Verfahren nach Anspruch 1 oder 2, bei dem die Lösung auf einer Temperatur von 25 bis 75° gehalten wird.

4. Verfahren nach einem der Ansprüche 1 bis 3, bei dem die aufkonzentrierte Lösung zu N-Methylmorpholin-N-Oxid weiterverarbeitet wird.

5. Verfahren nach einem der Ansprüche 1 bis 4, dadurch gekennzeichnet, daß die aufzukonzentrierende Lösung zusätzlich Avivage enthält.

## Claims

1. A process for separating water from a diluted aqueous solution of N-methylmorpholine-N-oxide, N-methylmorpholine, morpholine or mixtures thereof occurring in the production of cellulose shaped bodies by means of the solvent N-methylmorpholine-N-oxide, in which the solution is pressed through a semipermeable membrane in a reverse osmosis installation at a pressure which is greater than the osmotic pressure.

2. A process according to Claim 1, in which the solution is pressed through the semipermeable wall at a pressure of 40 bar.

3. A process according to Claim 1 or 2, in which the solution is kept at a temperature of 25 to 75°.

4. A process according to one of Claims 1 to 3, in which the concentrated solution is processed to N-methylmorpholine-N-oxide.

5. A process according to one of Claims 1 to 4, characterised in that the solution which is to be concentrated contains, in addition, freshener.

## Revendications

1. Procédé de séparation d'eau à partir d'une solution aqueuse diluée de N-méthylmorpholine-N-oxyde, N-méthylmorpholine, morpholine ou de leurs mélanges, apparaissant lors de la fabrication de corps moulés cellulosiques à l'aide de N-méthylmorpholine-N-oxyde comme solvant, dans lequel on force la solution à passer à travers une membrane semi-perméable dans une installation d'osmose inverse à une pression qui est supérieure à la pression osmotique.

2. Procédé selon la revendication 1, dans lequel on fait passer la solution à travers la membrane semi-perméable à une pression de 40 bar.

3. Procédé selon la revendication 1 ou 2, dans lequel la solution est maintenue à une température comprise entre 25°C et 75°C.

4. Procédé selon l'une quelconque des revendications 1 à 3, dans lequel la solution concentrée est ensuite transformée en N-méthylmorpholine-N-oxyde.

5. Procédé selon l'une quelconque des revendications 1 à 4, caractérisé en ce que la solution concentrée contient en outre de l'avivage.
